# EUROPEAN PATENT SPECIFICATION

(11) **EP 2 019 869 B1**
(45) Date of publication and mention of the grant of the patent: **02.03.2011**
(21) Application number: 07725429.0
(22) Date of filing: 22.05.2007
(51) Int. Cl.: C12P 17/02

(54) **FERMENTATIVE PRODUCTION OF LIPSTATIN**
FERMENTATIVE PRODUKTION VON LIPSTATIN
PRODUCTION DE LIPSTATINE PAR FERMENTATION

(30) Priority: 22.05.2006 EP 06010470
(43) Date of publication of application: 04.02.2009
(73) Proprietor: KRKA, 8501 Novo mesto (SI)
(72) Inventor: GASPARIC, Ales, 8000 Novo Mesto (SI); SLADIC, Gordan, 8000 Novo Mesto (SI); BENICKI SVAGELJ, Neda, 1000 Zagreb (HR); PELKO, Mitja, 8000 Novo Mesto (SI); RASPOR, Peter, 1000 Ljubljana (SI); PETKOVIC, Hrvoje, 1000 Ljubljana (SI); FUJS, Stefan, 9201 Puconci (SI)
(74) Representative: Becker Kurig Straus
(86) International application number: PCT/EP2007/004529
(87) International publication number: WO 2007/134836

(56) References cited:
- EP-A- 0 803 576
- WO-A-03/048335

## Description

The present invention pertains to the production of lipstatin. The present invention describes a process allowing the production of said enzyme inhibitor in high yields and high purity.

Obesity is a condition affecting more than 30% of the adult population in the industrial world and represents a major public health problem, since the excessive accumulation of body fat may be dangerous and leads to different health problems. Examples of obesity linked diseases are type II diabetes, hypertension, hyperlipidemia, coronary heart disease, stroke, breast and colon cancer, sleep apnea, gallbladder disease, gastroesophogeal reflux disease, fatty liver, gout, thromboembolism. Obesity is one of the main sectors of cardiovascular diseases. The levels of cholesterol, blood pressure, blood sugar and uric acid in obese people are usually higher than those of persons of normal weight. The morbidity from coronary heart disease among the overweight people is increased as well.

Determinants of obesity include social factors, psychological factors, genetic factors, developmental factors, and decreased physical activity. Components of a comprehensive weight loss program include medical assessment, behaviour and dietary modification, nutrition education, cognitive restructuring, increased physical activity, and long term follow-up.

Current medications directed to weight loss may be divided into two groups depending on their mechanisms of action: those affecting the central nervous system, and those that do not affect the central nervous system. The first type may reduce weight by affecting certain neurotransmitters to decrease appetite, but includes side effects such as a change in blood pressure or heart rate even when taken for a short term. The second type is a lipase depressant, which affects weight by reducing the absorption rate of fat by inhibiting lipases. These enzymes are capable to hydrolyze triglycerides into glycerol and free fatty acids in a process called lipolysis. The molecules obtained (primarily the free fatty acids) serve as blood-borne energy carriers which may be used by the liver, skeletal muscles, and other organs for aerobic respiration.

Examples for the second type of treatment of obesity with lipase inhibitors comprise the use of compounds such as lipstatin or lipstatin derivatives.

Lipstatin is a potent inhibitor of pancreatic lipase and a precursor for orlistat known from EP 0 129 748. Lipstatin contains a beta-lactone ring, probably accounting for the irreversible lipase inhibition, which carries two aliphatic residues with chain lengths of 6 and 13 carbon atoms. One of the side chains contains two isolated double bonds and a hydroxy group esterified to N-formyl-leucine.

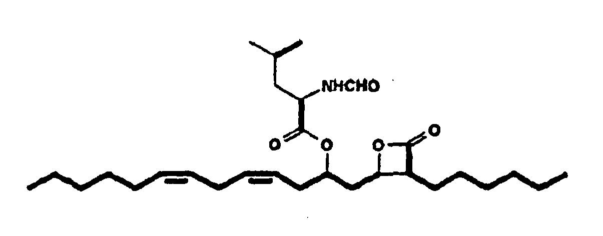

It was suggested that lipstatin may be assembled via Claisen condensation of octanoyl-CoA with 3-hydroxy-tetradecanoyl-CoA formed from linoleic acid. The tetrahydro-derivative of lipstatin, tetrahydrolipstatin (Orlistat^{®}, Xenical), is used for the treatment of severe obesity in forming a covalent adduct with a serine moiety of human pancreatic lipase by transesterification. Lipstatin and derivatives thereof act by induction of an increased fat excretion via the faeces (steatorrhoea), resulting in a lower bioavailability of fat ingested.

The chemical synthesis of the lipstatin derivative tetrahydrolipstatin is for example described by Hanessian, S. et al. (J. Org. Chem., 58 (1993), p. 7768-81) employing several synthesis steps with an overall yield of 38%.

Complex secondary metabolites such as lipstatin, however, are advantageously produced by biochemical processes using suitable strains. An example of such a strain capable of producing lipstatin is Streptomyces toxytricini (Weibel E. et al., J. Antibiot.(1987).p. 1081).

The respective underlying biosynthetic pathway may be pointed up by the following retro-biosynthetic illustration (1: lipstatin; 2: 3-hydroxy-Δ5,8-tetradecadienoyl-CoA; 3: octanoyl-CoA and 4: linoyl-CoA).

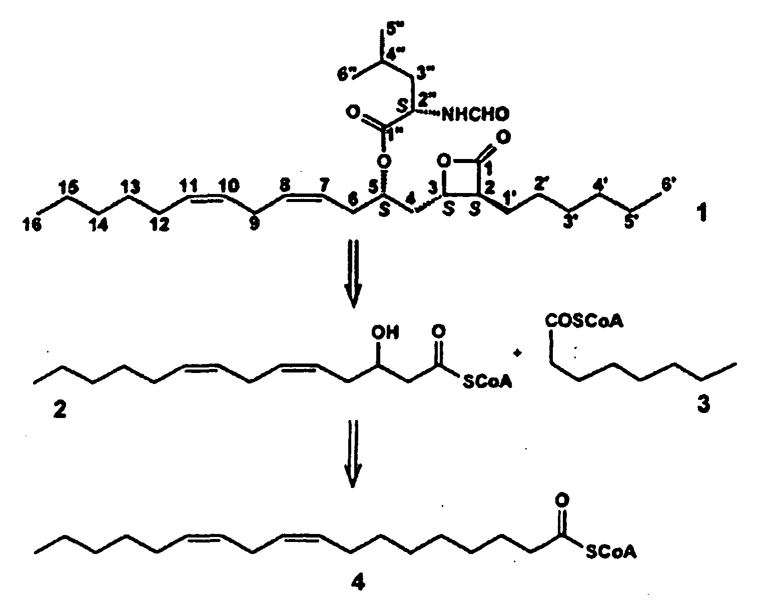

The production of lipstatin by biochemical methods such as a fermentation process is disclosed for example in EP 0 129 748. Drawbacks of the fermentation process as described in said reference are that lipstatin is contained in small amounts of few milligrams per litre rendering a subsequent isolation and purification difficult and complex.

EP 0 803 576 (Roche) disclosed a process wherein selected lipstatin precursors, namely linoleic acid, caprylic acid and N-formyl-L-leucine or preferably L-leucine, were inoculated into the fermentation. The yield of the fermentation was low because of the toxicity of the fatty acids and the amounts of feed solutions were very small. The feeding of the linoleic acid and caprylic acid and/or their salts was preferably conducted so that their concentration in the broth remained inferior to 1000 mg per liter. This fermentation process uses a medium that is substantially free of fats and oils because they result in uncontrolled fatty acid liberation during the fermentation and high residue at the end of the fermentation.

WO 03/048335 (Teva), disclosed a process for producing lipstatin based on fermentation medium comprising an oil and an assimilable carbon source, wherein w/w ratio of oil and assimilable carbon source is adjusted to regulate lipstatin biosynthesis by microorganism and introducing an emulsifier to regulate the fermentation broth viscosity. The preferred quantity of oil fed into the fermentation broth is not less than 5 % w/w. The use of the proposed amount of oil may results in higher amount of oil residues at the end of the process, which may be disadvantageous for further down-stream processing. Further more, according to the disclosed process a mixing rate was a key factor among the main cultivation parameters and preferably increased during the cultivation to compensate the decrease in dissolved oxygen level.

The objective of the present invention resides, therefore, in the provision of an improved process for the production of lipstatin in yields, allowing a reasonable isolation. Another objective resides in the provision of higher yields of lipstatin.

In the course of the extensive studies leading to the present invention the named inventors carried out experiments with chemical, synthesis and fermentative processes, eventually finding a fermentation process, which solves the above problem. Said process comprises the steps of a process for the production of lipstatin, comprising the steps of providing a medium comprising not less than 0.3 % w/w a linoleic acid or its ester(s) or salt(s) and more than 1 % w/w of oil inoculating the medium with a seed culture comprising a lipstatin producing microorganism; followed by cultivation; optionally isolation of lipstatin; In said process according the present invention, the main cultivation step, i.e. the proper fermentation step directed to the production of lipstatin, is performed with a medium comprising linoleic acid or its ester(s) or salt(s) and an oil in particular amounts.

Linoleic acid is, like some other unsaturated fatty acids, known in the art to exhibit a high toxicity towards the production strain. This effect is due to a production strain mediated epoxidation of a linoleic acid double bound by enzymes, such as a P450 monooxygenase, which enzyme converts the double bound to a high toxic epoxide. Toxic levels of linoleic acid are reported to be in the range of from 0.23 to 0.3 % w/w for the commonly used production strain, Streptomyces toxytricini.

The present inventors have in spite of this known effect unexpectedly found that by providing a medium for the main cultivation step comprising amounts of linoleic acid in a range supposedly toxic for the production strains Streptomyces toxytricini or Streptomyces virginiae, i.e. more than 0.3 % w/w linoleic acid and at least 1 % w/w of an oil, which oil may even contain additional amounts of linoleic acid, higher yields of lipstatin are obtained.

In said process according the present invention, the main cultivation step, i.e. the proper fermentation step directed to the production of lipstatin, could be performed at the conditions of limited oxygen supply, i.e. micro-aerophilic conditions, without the need to increase the mixing rate regulating the dissolved oxygen level higher than 10 %.

Without wishing to be bound by any theory it is presently assumed that the concurrent use of oil and linoleic acid has a combinatorial effect eventually resulting in a reduced toxicity of linoleic acid for the strain used to produce lipstatin. This allows conducting the main cultivation step in presence of amounts of linoleic acid otherwise being toxic. The lipstatin precursor linoleic acid will in turn at least partially metabolized by the production strain to lipstatin leading to higher yields of said lipase inhibitor.

According to the present invention, a process for the production of lipstatin is provided. Said process comprises the steps of (i) providing a medium having not less than 0.3 % w/w a linoleic acid or its ester(s) or salt(s) and more than 1 % w/w of oil, (ii) adding a seed culture comprising a lipstatin producing microorganism and at least 0.2% w/w of linoleic acid or its ester(s) or salt(s), (iii) cultivating and (iv) optionally isolating lipstatin.

It will be appreciated that the present process may be conducted in any kind of fermentation vessel, such as a sterile flask or fermentor.

The medium may be any kind of medium suitable to promote growth of microorganisms. The medium may be a synthetic medium, i.e. containing solely defined chemical compounds, or a complex medium, i.e. a medium comprising at least one constituent of a non-defined chemical composition, such as yeast extract or peptone. As used herein the expression "chemically defined medium" denotes a medium that essentially does not contain undefined nitrogen or carbon sources such as animal or plant protein or protein hydrolysate compositions or complex carbon sources such as e. g. molasses or corn steep liquor, but wherein the nitrogen sources are well-defined inorganic or organic compounds such as ammonia or amino acids and the carbon source is a well-defined sugar such as glucose. Additionally, such a synthetic medium contains mineral components such as salts, e. g. sulfates, acetates, phosphates and chlorides of alkaline and earth alkaline metals, vitamins and micronutrients. Examples of chemically defined media for the purpose of the present invention are given herein. It will be appreciated that these media are only examples. The person of skill in the art will be able to provide other media which permit cultivation of recombinant lactic acid bacteria under the above cultivation conditions.

The medium comprises a suitable carbon source and a suitable nitrogen source providing carbon- and nitrogen-containing compounds to the microorganism in such a way that the microorganism may use/convert these compounds for growth/development/reproduction and production of secondary metabolites, representing compounds of metabolism that are not essential for normal growth, development or reproduction of said microorganism.

Any kind of sugars and/or carbohydrates may serve as the carbon source, such as, for example, glucose, saccharose, lactose, fructose, maltose, molasses, starch and cellulose. Alternatives for the carbon source are oils and fats, such as, for example, soybean oil, sunflower oil, groundnut oil and coconut oil, or fatty acids, such as, for example, palmitic acid, stearic acid and linoleic acid, or alcohols, such as, for example, glycerol and ethanol, and/or organic acids, such as, for example, acetic acid. Those substances may be used individually or in the form of a mixture.

As the nitrogen source any organic nitrogen-containing compound(s) is(are) contemplated, such as peptones, yeast extract, meat extract, malt extract, corn steep liquor, soybean flour and urea, or inorganic compounds, such as ammonium sulfate, ammonium chloride, ammonium phosphate, ammonium carbonate and ammonium nitrate. The nitrogen source(s) may be used individually or in the form of a mixture. The nitrogen source(s) may be also be added in a form rendering it suitable for controlling the pH of the reaction vessel.

The concentrations of carbon source and nitrogen source depend on the type of microorganism and the selected cultivation conditions. The carbon source and/or the nitrogen source in the culture is/are kept at a pre-selected concentration of at least about 0.5 g/l by controlled feeding of the respective compound(s) in a fed-batch process or by feeding of a solution in a continuous cultivation process. The concentration of the carbon and independently therefrom the nitrogen source in the cultivation medium may preferably be at least 5 g/l, more preferably at least 10, 15, 20, 30, 40, 50, 80 or 100 g/l.

The culture medium may also contain a phosphorus source, e.g. phosphoric acid, potassium dihydrogen phosphate or dipotassium hydrogen phosphate or the corresponding sodium-containing salts and/or metal salts, such as, for example, magnesium sulfate or iron sulfate, which are necessary for growth. Essential growth substances, such as amino acids and vitamins, may be also used in addition to the above-mentioned substances. Suitable precursors, particularly for the secondary metabolite to be produced, may also be added to the culture medium.

The above substances may be added to the culture in the form of a single batch, or they may be fed in a suitable manner during the cultivation. The medium may comprise other compounds such as vitamins, emulsifiers and antifoam. Also, the above compounds may be present in the respective required concentration in the medium. Descriptions of culture media for various microorganisms are to be found in the handbook "Manual of Methods for General Bacteriology" of the American Society for Bacteriology (Washington D.C., USA, 1981) or in case of Actinomycetes or Streptomyces in the handbook "Practical Streptomyces genetics" by Kieser, T., Bibb, M.J., Chater, K.F., Hopwood, D.A., (2000), John Innes Foundation, Norwich, United Kingdom.

The amount/feed of carbon source, nitrogen source or any other suitable constituents of the medium may be controlled by linking or connecting such feeding to any sensor means capable of detecting conditions, such as pH or concentration of secondary metabolite, in the bioreactor. In this manner, feeding of e.g. glucose or a lipstatin precursor is activated by the means controlling the automatic addition said compounds. Such means may be employed in a continuous process as well.

In order to control the pH value of the culture, basic compounds, such as sodium hydroxide, potassium hydroxide, ammonia or ammonia water, or acid compounds, such as phosphoric acid or sulfuric acid, are expediently used. In order to control the development of foam, anti-foams, such as, for example, fatty acid polyglycol esters, may be used. In order to maintain aerobic conditions, if desired, oxygen or gas mixtures containing oxygen, such as, for example, air, are introduced into the culture. The temperature of the culture is normally from 20 to 40°C, preferably 28 to 32°C. The culture is usually continued until the maximum amount of the desired product has formed, which may be achieved within a time period of from about 10 hours to about 200 hours.

The seed or pre-culture may comprise essentially the same constituents as the medium. In some cases, however, it may be desired that the production or overproduction of the secondary metabolite, which represents a stress situation for the microorganism and consequently reduces essential vital functions, starts in the main culture. In such a case the medium composition of main and seed culture may be different.

The lipstatin producing microorganism may be any microorganism capable of producing said compound including also genetically modified microorganisms. An example of a lipstatin producing microorganism is Streptomyces toxytricini (Weibel E. et al.. J. Antibiot. (1987), p. 1081). There are, however, other Streptomycetes or Actinomycetes having the capability to produce the same compound.

In a preferred embodiment, the production strains comprise Streptomyces toxytricini CBS 314.55, CBS 566.68, ATCC 19813, ATCC 19928 and Streptomyces virginiae CBS 314.55, which strains were either obtained by the American Type Culture Collection (ATCC; USA) or by the Cantraalbureau for Schimmelcultures (CBS, Netherlands).

Cultivating is directed to any cultivation of microorganism using commonly known processes, including chemostat, batch, fed-batch cultivations, etc. at suitable conditions favouring growth and/or production of a secondary metabolite.

Respective methods for isolating and purifying secondary metabolites, such as lipstatin, from fermentation broths are well known to the skilled person. Extractions may be e.g. performed in aqueous solution with suitable organic solvents and is described e.g. in EP 1 028 115.

Methods of determining lipstatin or derivatives thereof are known in the art. The analysis may be carried by e.g. reversed phase HPLC using an appropriate column and running conditions (Inertsil C-18, mobile phase: acetonitrille:H3PO4, detection 200 nm).

The medium contains not less than 0.3 % w/w linoleic acid and more than 1 % w/w of an oil. The oil may be any oil from synthetic or natural origin or a mixture thereof, which compositions and preparation are known to the skilled person. Examples for suitable oils are soya bean oil, palm oil, sunflower oil, flax oil, rape seed oil and corn germ oil.

Preferably, more than 0.3 % w/w up and not more than 10 % w/w linoleic acid are included. Independently from the amount of linoleic acid, the amount of the oil may be varied. Preferably, more than 1 % w/w and not more than 5 % w/w of the oil are included.

According to a preferred embodiment of the present invention the carbon source is a sugar selected from the group consisting of glucose, fructose and glycerol. Preferably, glycerol is employed as the carbon source. The glycerol concentration in the cultivation medium is preferably at least 5 g/l, more preferably at least 10, 15, 20, 30, 40, 50, 80 or 100 g/l.

According to another embodiment the nitrogen source is yeast extract or peptone. Preferably, yeast extract is employed as nitrogen source. The concentration of yeast extract in the cultivation medium is preferably at least 1 g/l, more preferably at least 2, 3, 4, 5, 10, 15, 20 or 25 g/l.

According to still another embodiment the lipstatin producing microorganism is Streptomyces toxytricini. Suitable medium compositions and growth conditions for said strain may be for example found in the handbook "Practical Streptomyces genetics".

The seed culture itself comprises at least 0.2 % w/w of linoleic acid, which has been surprisingly found by the present inventors to exert a boosting effect on the amounts of lipstatin obtained during the main culture. Preferably, an amount at least 0.25 % w/w of linoleic acid is used in the seed culture, more preferably at least 0.3, 0.35, 0.4, 0.45 or 0.5 % w/w of linoleic acid.

The present inventors have found that the step of the main culture may also be performed under anaerobic conditions yielding even higher amounts of lipstatin. This represents a surprising finding due to the fact, that Streptomyces toxytricini and other lipstatin producing Actinomycetes are strict aerobic microorganisms requiring a minimal amount of oxygen for growth. According to still another preferred embodiment, the step of cultivating is performed under anaerobic conditions.

According to another embodiment of the present invention, leucine is added. Addition of said compound, as a precursor of lipstatin bisoynthesis, improves yields since the production of impurities such as methionine Lipstatin is reduced. Preferably, more than 10⁻⁴ % w/w up and not more than 2.0 % w/w leucine are included. It has been found particularly suitable to add leucine in a feeding process having a feed rate of between 10⁻⁴ % w/w and 10⁻² % w/w leucine per hour. It will be appreciated that leucine as well as any other compound fed may be in pure form or alternatively diluted with e.g. water.

The present inventors have also found that addition of the nitrogen source in form of an organic or inorganic salt of defined, i.e. known, composition has a advantageous impact on the yields. Without wishing to be bound by any theory it is assumed that the so called complex compounds such as peptone and yeast extract comprise ingredients inhibiting the biosynthesis of lipstatin to a certain extent. An example for an organic or inorganic salt of a defined composition is an ammoniumsalt such as ammoniumsulfate, which has been found particularly suitable, since on the one hand yields of lipstatin are increased and on the other hand ammoniumsulfate may be used to control the pH in the reaction vessel. Best results could be obtained by addition of leucine as well as an organic or inorganic salt of a defined composition.

This issue further suggests that in general the addition of any complex compound (of an unknown composition) should be avoided in that according to another preferred embodiment none of the consituents used for the present process has a complex, i.e. undefined, composition. Examples for suitable medias of such a known composition are within the knowledge of the skilled person.

According to an embodiment of the present invention, lipstatin may be converted to a derivative thereof in a manner known in the art. A suitable derivative is for example tetrahydrolipstatin. Alternatively, any pharmaceutical or nutritional acceptable salt of lipstatin, tetrahydrolipstatin or other lipstatin derivative is encompassed by the term "derivative thereof". Preferably, the lipstatin derivative is tetrahydrolipstatin.

According to an embodiment, said oil is a natural oil selected from the group consisting of soya bean oil, palm oil, sunflower oil, flax oil, rape seed oil and corn germ oil or a mixture thereof. Preferably, soya bean oil is employed.

The following examples illustrate the invention without limiting it thereto.

### Examples

### Example 1.

### a.) Seed culture Process

The composition of the seed culture medium was as follows:

| | |
|---|---|
| soya bean flour | 10 g |
| glycerol | 20 g |
| yeast extract | 5 g |
| tap water | 1 l |

The pH was adjusted to 7 with NaOH 10 %. 5 ml of this medium was filled into a 50 ml tube, closed with a foam plug and sterilised at 121°C for 20 minutes. The sterilized inoculum medium was inoculated with a spore suspension of Streptomyces toxytricini and incubated at 28°C for 20-30 hours under aerobic conditions.

### b.) Main Fermentation Process

About 10 % v/v of the above seed culture was used for the inoculation of a 50 ml tube, which contained 5 ml of fermentation medium.

The composition of the fermentative medium was as follows:

| | |
|---|---|
| soya bean flour | 32 g |
| glycerol | 20 g |
| lecithin | 14.0 g |
| CaCO₃ | 2.0 g |
| MES | 2 g |
| soya bean oil | 10 g |
| linoleic acid | 30 g |
| tap water | 1 l |

The pH of the fermentation medium was adjusted before sterilization to pH 7.4 with 10% NaOH. Sterilization was performed at 121°C for 20 minutes. Fermentation was carried out at 28°C for 6-7 days under aerobic conditions.

After fermentation for 144 hours, the concentration of lipstatin was 417±55 mg/l

### Example 2.

Example 1 was repeated with the exception that the 2 g of linoleic acid was added to the composition of the seed culture medium.

After fermentation for 144 hours, the concentration of lipstatin was 556±81 mg/l

### Example 3.

### a.) Seed culture Process

A seed culture process was repeated as in Example 1 with the exception that the inoculum medium was inoculated with a spore suspension of Streptomyces virginiae.

### b.) Main Fermentation Process

About 10 % v/v of the above seed culture was used for the inoculation of a 40 ml o fermentation medium dispensed in 500 ml Erlenmeyer flask.

The composition of the fermentative medium was as follows:

| | | | |
|---|---|---|---|
| soya bean flour | 40 g | 40 g | 40 g |
| glycerol | 20 g | 20 g | 20 g |
| lecithin | 14.0 g | 14.0 g | 14.0 g |
| yeast Extract | 5.0 g | 5.0 g | 5.0 g |
| soya bean oil | / | 10 g | 10 g |
| linoleic acid | 20 g | 20 g | / |
| tap water | 1 l | 1 l | 1 l |
| Titer of lipstatin after 6 days of fermentation | 5 mg/l | 67 mg/l | 34 mg/l |

The pH of the fermentation medium was adjusted before sterilization to pH 7.2 with 10% NaOH. Sterilization was performed at 121°C for 20 minutes. Fermentation was carried out at 28°C for 6 days under aerobic conditions.

### Example 4.

### Lipstatin production in 150 l bioreactor

### a.) Seed culture process

The composition of the seed culture medium was as follows:

| | |
|---|---|
| soya bean flour | 10 g |
| glycerol | 10 g |
| yeast extract | 5 g |
| Antifoam agent | 10 ml |
| tap water | 1 l |

Seed culture medium was prepared in bioreactor with 30 l working volume. The pH was adjusted to 7.0 with 10% NaOH, sterilized for 20 minutes at 121 °C and than cooled to 27°C.

Seed culture medium was inoculated with 150 ml of spore suspension. Stirrer speed was set to 250 rpm and the aeration rate was 15 1 per minute. In 22 to 30 hours the packed cell volume was more than 12%.

### b.) Main Fermentation Process

About 10 I of this seed culture was used to inoculate a fermentor with a vessel size of 150 l containing 90 I of a production medium containing:

| | |
|---|---|
| soya bean flour | 30 g |
| glycerol | 20 g |
| lecithin | 14.0 g |
| CaCO₃ | 2.0 g |
| NaCl | 0.7 g |
| Na₂SO₄ | 2 g |
| KH₂PO₄ | 0.1 g |
| MnCl₂ x 4 H₂O | 0.33 g |
| MgSO₄ x 7 H₂O | 2.0 g |
| soya bean oil | 10 g |
| linoleic acid | 10 g |
| antifoam agent | 0.5 ml |
| tap water | 1 l |

Before sterilization pH was adjusted to 7.0 with NaOH 10 % and subsequently sterilized for 30 minutes at 121°C.

Temperature of fermentation was set to 27°C, stirrer speed was set to 350 rpm, aeration rate was 100 1 per minute. pH was controlled by addition of 10% NaOH and 30% H₂SO₄ and maintained between 7.1 and 6.8. Minimal concentration of dissolved oxygen was set to 30% after reaching that level, stirrer speed was gradually increased to a maximal speed of 450 rpm.

20 hour after inoculation feeding with linoleic acid was started. The rate of feeding was controlled in such way that the level of linoleic acid is slightly increased. From 70 to 100 hours after inoculation the concentration of dissolved oxygen dropped to 0% but surprisingly the production of lipstatin continued with unchanged rate. Besides that during fermentation the level of linoleic acid was higher than 0.3 % what was previously known as toxic level but such high concentration did not have any influence on lipstatin production. At the end of fermentation (209 hour after inoculation ), titre of lipstatin was 914 mg/l.

**Table 2: Titre of lipstatin in fermentation in 150 l bioreactor.**

| Hours after inoculation | Titre of lipstatin (mg/l) |
|---|---|
| 41 | 187 |
| 65 | 274 |
| 89 | 528 |
| 113 | 611 |
| 137 | 774 |
| 161 | 724 |
| 185 | 870 |
| 209 | 914 |

### Example 5.

Seed culture process and medium preparation of main fermentation process were repeated as described in Example 4 with the exception that the medium of the main fermentation process comprise 10 g of glycerol instead of 20 g of glycerol as in Example 4.

Temperature of fermentation was set to 27°C, stirrer speed was limited to 330 rpm, aeration rate was up to 50 1 per minute. pH was controlled with the addition of 10% NaOH and 30% H₂SO4 and maintained between 7.1 and 6.8. Within 24 hours concentration of dissolved oxygen dropped to 1%. After reaching that level stirrer speed was regulated to keep the oxygen concentration at about 1%. Feeding with linoleic acid was started twenty hours after inoculation. The rate of feeding was controlled as in example 4. At the end of fermentation (160 hour after inoculation) titre of lipstatin was 1500 mg/l.

**Table 3: Titre of lipstatin and concentration of linoleic acid in fermentation in 150 l bioreactor**

| Hours after inoculation | Titre of lipstatin (mg/l) | Concentration of linoleic acid (g/l) |
|---|---|---|
| 0 | p.m.z. | 1,3 |
| 17 | 44 | 0.4 |
| 41 | 289 | 0,9 |
| 67 | 674 | 0,9 |
| 90 | 808 | 1,4 |
| 113 | 1092 | 1,6 |
| 137 | 1386 | 2 |
| 161 | 1476 | 2,4 |
| 185 | 1575 | 3 |

### Example 6.

After the completion of the fermentation process, the fermentation broth was acidified with a sulphuric acid to the pH value 1.6 ± 0.1. Mycelium was separated and an amount of 2 kg of wet cake containing 9.2 g / kg of lipstatin was extracted with 10 1 of acetonitrile. Solvent was separated and the lipstatin containing oily substance was concentrated by vacuum evaporation at 40°C. The 74 g of the oily substance containing 12.8 g of lipstatin was obtained.
An amount of 37 g of the obtained oily substance containing 6.4 g of lipstatin was purified at the silicagel column (40 - 63 µm particle size) and with tert-butyl methyl ether:heptan (ratio 2:3) as a mobile phase. After the evaporation of the mobile phase the 5.03 g of 93 % lipstatin (determined by HPLC) was obtained.

### Example 7.

### Feeding with linoleic acid and ammonia sulphate

### Lipstatin production in 150 l bioreactor.

Seed culture medium of following composition was prepared in bioreactor with 30 1 working volume: 350 g of soy bean flour, 350 g of glycerol, 175 g of yeast extract, 70 ml of linoleic acid, 35 ml antifoam agent and tap water to 35 1. The pH was adjusted to 6.5 with 30% H₂SO₄. Sterilisation was performed for 20 minutes at 121°C and subsequently the vessel was cooled to 27°C. Seed culture medium was inoculated with 150 ml of spore suspension. Stirrer speed was set to 200 rpm and the aeration rate was 10 1 per minute. In 22 to 30 hours the packed cell volume was more than 12%.

A volume 101 of this seed culture was used to inoculate a fermentor with a vessel size of 150 l containing 90 1 of a production medium containing: 4 kg of soya bean flour, 1 kg of glycerol, 1.4 kg of lecithin, 50 ml of antifoam agent, 200 g of CaCO₃, 1000 g of soya bean oil, 200 g of linoleic acid, 8 g of FeSO₄, 4 g of ZnSO₄, 12 g of MnCl₂ x 4 H₂O and 12 g of MgSO₄ x 7 H₂O. Before sterilization pH was adjusted to 6.5 with 30% H₂SO₄ and subsequently sterilized for 30 minutes at 121°C. Temperature of fermentation was set to 27°C, stirrer speed was limited to 330 rpm, aeration rate was up to 50 1 per minute. pH was controlled with the addition of 25% NH₃ and 30% H₂SO₄ and maintained between 7.1 and 6.8. Within 24 hours concentration of dissolved oxygen dropped to 1%. After reaching that level stirrer speed was regulated to keep the oxygen concentration at about 1%. Feeding with linoleic acid and 30% solution of ammonium sulphate was started twenty hours after inoculation. The rate of feeding of linoleic acid was controlled as in example 5. The rate of feeding of 30% solution of ammonium sulphate was up to 12 g/h till the end of fermentation. At the end of fermentation (130 hour after inoculation) titre of lipstatin was 1571 mg/l.

Solution of ammonium or ammonium salt was used as a supplement of nitrogen. A constant source of easily metabolizable nitrogen reduced the rate of amino acid degradation. With the rate of ammonia addition in combination with fermentation process parameters pH values were regulated.

**Table: Titre of lipstatin and concentration of linoleic acid in fermentation in 150 l bioreactor**

| Hours after inoculation | Titre of lipstatin (mg/l) |
|---|---|
| 18 | 198 |
| 40 | 586 |
| 59 | 830 |
| 76 | 910 |
| 80 | 994 |
| 102 | 1240 |
| 130 | 1571 |

### Example 8.

### Feeding with linoleic acid and leucine

### Lipstatin production in 150 l bioreactor

Seed culture medium of following composition was prepared in bioreactor with 30 1 working volume: 350 g of soya bean flour, 350 g of glycerol, 175 g of yeast extract, 70 ml of linoleic acid, 35 ml antifoam agent and tap water to 35 1. The pH was adjusted to 7.0 with 10% NaOH. Sterilisation was performed for 20 minutes at 121 °C and subsequently the vessel was cooled to 27°C. Seed culture medium was inoculated with 150 ml of spore suspension. Stirrer speed was set to 200 rpm and the aeration rate was 10 1 per minute. In 22 to 30 hours the packed cell volume was more than 12%.

A volume 10 I of this seed culture was used to inoculate a fermentor with a vessel size of 150 1 containing 90 1 of a production medium containing: 4 kg of soya bean flour, 1 kg of glycerol, 1.4 kg of lecithin, 50 ml of antifoam agent, 200 g of CaCO₃, 1000 g of soya bean oil, 200 g of linoleic acid, 8 g of FeSO₄, 4 g of ZnSO₄, 12 g of MnCl₂ x 4 H₂O and 12 g of MgSO₄ x 7 H₂O. Before sterilization pH was adjusted to 6.5 with NaOH 10 % and subsequently sterilized for 30 minutes at 121°C. Temperature of fermentation was set to 27°C, stirrer speed was limited to 330 rpm, aeration rate was up to 50 1 per minute. pH was controlled with the addition of 10% NaOH and 30% H₂SO₄ and maintained between 7.1 and 6.8. Within 24 hours concentration of dissolved oxygen dropped to 1%. After reaching that level stirrer speed was regulated to keep the oxygen concentration at about 1%. Feeding with linoleic acid and 10% solution of leucine was started twenty hours after inoculation. The rate of feeding of linoleic acid was controlled as in example 5. The rate of feeding of 10% solution of leucine was up to 25 g/h till the end of fermentation. At the end of fermentation (160 hour after inoculation) titre of lipstatin was 3249 mg/l. Addition of leucine during fermentation had major effect on reduced synthesis of one of main lipstatin related impurities - methionine lipstatin.

**Table: Titre of lipstatin and concentration of linoleic acid in fermentation in 150 l bioreactor**

| Hours after inoculation | Titre of lipstatin (mg/l) |
|---|---|
| 22 | 212 |
| 44 | 795 |
| 59 | 1187 |
| 80 | 1877 |
| 98 | 2245 |
| 118 | 2782 |
| 140 | 2928 |
| 164 | 3249 |

### Example 9.

### Feeding with linoleic acid and leucine and ammonia sulphate

### Lipstatin production in 150 l bioreactor

Seed culture medium of following composition was prepared in bioreactor with 30 1 working volume: 350 g of soya bean flour, 350 g of glycerol, 175 g of yeast extract, 70 ml of linoleic acid, 35 ml antifoam agent and tap water to 35 1. The pH was adjusted to 6.5 with 30% H₂SO₄. Sterilisation was performed for 20 minutes at 121°C and subsequently the vessel was cooled to 27°C. Seed culture medium was inoculated with 150 ml of spore suspension. Stirrer speed was set to 200 rpm and the aeration rate was 10 1 per minute. In 22 to 30 hours the packed cell volume was more than 12%.

A volume 10 l of this seed culture was used to inoculate a fermentor with a vessel size of 150 1 containing 90 1 of a production medium containing: 4 kg of soya bean flour, 1 kg of glycerol, 1.4 kg of lecithin, 50 ml of antifoam agent, 200 g of CaCO₃, 1000 g of soya bean oil, 200 g of linoleic acid, 8 g of FeSO₄, 4 g of ZnSO₄, 12 g of MnCl₂ x 4 H₂O and 12 g of MgSO₄ x 7 H₂O. Before sterilization pH was adjusted to 6.5 with 30% H₂SO₄ and subsequently sterilized for 30 minutes at 121°C. Temperature of fermentation was set to 27°C, stirrer speed was limited to 330 rpm, aeration rate was up to 50 1 per minute. pH was controlled with the addition of 25% NH₃ and 30% H₂SO₄ and maintained between 7.1 and 6.8. Within 24 hours concentration of dissolved oxygen dropped to 1%. After reaching that level stirrer speed was regulated to keep the oxygen concentration at about 1%. Feeding with linoleic acid and nitrogen feeding solution (10% solution of leucine and 10% solution of ammonium sulphate) was started twenty hours after inoculation. The rate of feeding of linoleic acid was controlled as in example 5. The rate of feeding of nitrogen feeding solution was up to 25 g/h till the end of fermentation. At the end of fermentation (136 hour after inoculation) titre of lipstatin was 4773 mg/l.

**Table: Titre of lipstatin and concentration of linoleic acid in fermentation in 150 l bioreactor**

| Hours after inoculation | Titre of lipstatin (mg/l) |
|---|---|
| 16 | 74 |
| 40 | 1210 |
| 65 | 2012 |
| 89 | 2581 |
| 93 | 2698 |
| 117 | 3701 |
| 136 | 4773 |

### Example 10.

### Extraction of lipstatin

After the completion of the fermentation process, a volume of 2500 1 of fermentation broth (containing 1.9 g/l of lipstatin) was acidified with sulphuric acid (30 %) to the pH value 2.5 ± 0.5. Fermentation broth was charged into a reaction vessel and mixed with 1300 1 of ethyl acetate from previous extractions (containing 0.7 g/l of lipsatin) and 700 I of fresh ethyl acetate.

1^{st} Extraction: Mixture was fed into a continuous extractor operated at the feed rate of 2 m³/h. Additional 715 1 of fresh ethyl acetate was added as a counter-flow during the extraction. After the completion of extraction 1827 1 of ethyl acetate extract was obtained containing 4.0 kg of lipsatin, and 3450 l of extracted broth containing 1.9 kg of lipstatin.

2^{nd} Extraction: 3450 1 of extracted broth from the 1^{st} extraction was re-extracted with 1000 1 of fresh ethyl acetate. After the completion of the extraction 1383 1 of ethyl acetate extract was obtained, containing 1.3 kg of lipstatin and 3040 1 of extracted broth containing 0.7 kg of lipstatin.

Ethyl acetate extracts were joined to obtain 5.3 kg of lipstatin in ethyl acetate and further concentrated. Concentrate was re-extracted in acetonitrile twice (volume ratio of concentrate to acetonitrile was 1 : 3) and the undissolved impurities were separated. Acetonitrile phase was further concentrated to obtain crude lipstatin.

### Example 11.

### i. Chromatographic purification of lipstatin

Crude lipstatin was subjected to chromatography purification using silicagel as the stationary phase and mixture t-butyl methyl ether : heptane as a mobile phase.

### Chromatographic conditions

Column: 10 cm x 150 cm filed with 4000 g of silicagel 40 - 63 µm
Mobile phase: t-butyl methyl ether : heptane (volume ratio 40 : 60)
Load: mixture of silicagel and crude lipstatin (weight ration 10 : 1)
Flow rate: 50 ml/min

Fractions containing lipstatin were collected and lipstatin of 92.0 % purity was obtained.

### ii. Chromatographic purification of lipstatin

Crude lipstatin was subjected to chromatography purification using silicagel as the stationary phase and mixture t-butyl methyl ether : heptane as a mobile phase.

### Chromatographic conditions

Column: 8500 g of silicagel 40 - 63 µm
Mobile phase: t-butyl methyl ether : heptane (volume ratio 40 : 60)
Load: 580 ml of crude lipstatin (HPLC purity 14.8 %)
Flow rate: 30 ml/min

Fractions containing lipstatin were collected and 520 g lipstatin of 82.0 % purity was obtained.

### iii. Chromatographic purification of lipstatin

Crude lipstatin was subjected to chromatography purification using silicagel as the stationary phase and mixture t-butyl methyl ether : heptane as a mobile phase.

### Chromatographic conditions

Column: 8500 g of silicagel 40 - 63 µm
Mobile phase: t-butyl methyl ether : heptane (volume ratio 40 : 60)
Load: 1700 ml of crude lipstatin (HPLC purity 44.8 %)
Flow rate: 30 ml/min

Fractions containing lipstatin were collected and 630 g lipstatin of 74.0 % purity was obtained.

### Example 12.

### Preparation of orlistat by hydrogenation

Orlistat can be synthesized by hydrogenation of lipstatin as obtained after extraction or purification step.
Lipstatin, (35 g), ethanol (0,8 L) and 3,3 g of 5% Pd/C was charged in a reactor and stirred for a few hours at 10 to 40°C. Then the mixture is filtered and the clear solution was charged to an autoclave vessel and additional 3,3 g of 5% palladium on active carbon was added. Autoclave was closed, inertised by nitrogen and filled by hydrogen to 3.5 bar. The mixture was stirred at 15 to 25°C till the consumption of hydrogen was ceased. Then hydrogen is released and autoclave is vented by nitrogen. The catalyst is filtered off and the solvent is evaporated off. 34,7 g of slightly yellowish oil, which during the time converts to a waxy solid, was obtained.

### Example 13.

### Crystallization of orlistat

Orlistat obtained by hydrogentation (15 kg, assay 97,6%), a mixture of the form I and form II as described in WO 2005/026140, and heptane (60 L) are charged in an glass lined reactor and stirred by impeller stirrer. Suspension is dissolved by heating and then it is cooled to about 22°C, seeded by 10 g of orlistat form II, stirred at the same temperature for I hour and cooled to 15°C in about 2 hours. The obtained crystals are aged for 15 hours and filtered off by suction. After drying in vacuum, 14,1 kg of material is obtained. The XRPD of this material corresponds to orlistat form II, assay 100,4% using HPLC method.

## Claims

1. A process for the production of lipstatin, comprising the steps of:
- providing a medium having
a.) not less than 0.3 % w/w a linoleic acid or its ester(s) or salt(s) and
b.) more than 1 % w/w of oil
- inoculating the medium with a seed culture comprising a lipstatin producing microorganism and at least 0.2 % w/w of linoleic acid or its ester(s) or salt(s). ;
- cultivating and optionally isolating lipstatin.

2. The process according to claim 1, wherein the oil is selected from the group consisting natural oil, a synthetic oil and a mixture thereof.

3. The process according to claim 1, wherein the natural oil is selected from the group consisting soya bean oil, sunflower oil, repeseed oil, palm oil, flax oil, rape seed oil and corn germ oil or a mixture thereof.

4. The process of claim 1, wherein the lipstatin-producing microorganism is of the family of streptomycetes.

5. The process of claim 4, wherein the lipstatin-producing microorganism is Streptomyces toxytricini or Streptomyces virginiae.

6. The process of claim 5, wherein the lipstatin-producing microorganism is Streptomyces toxytricini CBS 566.68.

7. The process according to claim 1, wherein the pO₂ is regulated between 0 and 10 %, after the initial growth phase is substantially finished.

8. The process according to any of the preceding claims, wherein leucine and/or a nitrogen source in form of an organic or inorganic salt of a defined composition is added.

9. A process for the production of tetrahydrolipstatin, comprising production of lipstatin according to any of the preceding claims; and the step of converting Lipstatin to tetrahydrolipstatin.

## Patentansprüche

1. Verfahren zur Herstellung von Lipstatin, das die Schritte umfasst:
- Bereitstellen eines Mediums mit
a) nicht weniger als 0,3 % Gew./Gew. einer Linolensäure oder dessen Ester(n) oder Salz(en) und
b) mehr als 1 % Gew./Gew. Öl
- Inokulieren des Mediums mit einer Impfkultur, die einen Lipstatin produzierenden Mikroorganismus aufweist, und mindestens 0,2 % Gew./Gew. Linolensäure oder dessen Ester(n) oder Salz(en); und
- Züchten und wahlweise Isolieren von Lipstatin.

2. Verfahren nach Anspruch 1, wobei das Öl ausgewählt ist aus der Gruppe bestehend aus natürlichem Öl, einem synthetischem Öl und einem Gemisch davon.

3. Verfahren nach Anspruch 1, wobei das natürliche Öl ausgewählt ist aus der Gruppe bestehend aus Sojabohnenöl, Sonnenblumenöl, Rapssamenöl, Palmöl, Flachsöl, Rapssaatöl und Getreidekeimöl oder einem Gemisch davon.

4. Verfahren nach Anspruch 1, wobei der Lipstatin produzierende Mikroorganismus von der Familie der Streptomyceten ist.

5. Verfahren nach Anspruch 1, wobei der Lipstatin produzierende Mikroorganismus Streptomyces toxytricini oder Streptomyces virginiae ist.

6. Verfahren nach Anspruch 1, wobei der Lipstatin produzierende Mikroorganismus Streptomyces toxytricini CBS 566.68 ist.

7. Verfahren nach Anspruch 1, wobei der p02 zwischen 0 und 10 % liegt, nachdem die anfängliche Wachstumsphase im Wesentlichen beendet ist.

8. Verfahren nach einem der vorhergehenden Ansprüche, wobei Leucin und/oder eine Stickstoffquelle in Form eines organischen oder anorganischen Salzes einer bestimmten Zusammensetzung zugesetzt wird.

9. Verfahren zur Herstellung von Tetrahydrolipstatin, welches die Herstellung von Lipstatin nach einem der vorhergehenden Ansprüche umfasst, und den Schritt der Umwandlung von Lipstatin zu Tetrahydrolipstatin.

## Revendications

1. Un procédé pour la production de lipstatine comprenant les étapes de :
- fournir un milieu comportant
a.) pas moins de 0.3 % en poids d'acide linoléique ou de son (ses) ester(s) ou de son (ses) sel(s) et
b.) plus de 1 % en poids d'huile ;
- inoculer le milieu avec une culture d'ensemencement comprenant un microorganisme producteur de lipstatine et au moins 0.2 % en poids d'acide linoléique ou de son (ses) ester(s) ou de son (ses) sel(s) ;
- cultiver et le cas échéant isoler la lipstatine.

2. Le procédé selon la revendication 1 dans lequel l'huile est sélectionnée à partir du groupe consistant en huile naturelle, une huile synthétique et un mélange de celles-ci.

3. Le procédé selon la revendication 1 dans lequel l'huile naturelle est sélectionnée à partir du groupe consistant en huile de soya, huile de tournesol, huile de palme, huile de lin, huile de graine de colza, huile de germe de maïs ou un mélange de celles-ci.

4. Le procédé selon la revendication 1 dans lequel le microorganisme producteur de lipstatine est de la famille des streptomycètes.

5. Le procédé selon la revendication 1 dans lequel le microorganisme producteur de lipstatine est Streptomyces toxytricini ou Streptomyces virginiae.

6. Le procédé selon la revendication 1 dans lequel le microorganisme producteur de lipstatine est Streptomyces toxytricini CBS 566.68.

7. Le procédé selon la revendication 1 dans lequel la p02 est ajustée entre 0 et 10 % après que la phase initiale de croissance est substantiellement terminée.

8. Le procédé selon l'une des revendications précédentes dans lequel est ajouté de la leucine et/ou une source d'azote sous forme d'un sel organique ou inorganique d'une composition définie.

9. Un procédé pour la production de tétrahydrolipstatine comprenant la production de lipstatine selon l'une des revendications précédentes et l'étape de conversion de lipstatine en tétrahydrolipstatine
